(19) Europäisches Patentamt
European Patent Office
Office européen des brevets

(11) Publication number: **0 089 089**
**A1**

# (12) EUROPEAN PATENT APPLICATION

(21) Application number: 83200346.1

(22) Date of filing: 11.03.83

(51) Int. Cl.³: C 07 D 295/08
C 07 D 295/12, C 07 D 295/14
A 61 K 31/495

(30) Priority: 12.03.82 NL 8201032

(43) Date of publication of application: 21.09.83 Bulletin 83/38

(84) Designated Contracting States: AT BE CH DE FR GB IT LI LU NL SE

(71) Applicant: DUPHAR INTERNATIONAL RESEARCH B.V
C.J. van Houtenlaan 36
NL-1381 CP Weesp(NL)

(72) Inventor: Van Dalen-Van der Aa, Dirkje A.
c/o OCTROOIBUREAU ZOAN B.V. Apollolaan 151
NL-1077 AR Amsterdam(NL)

(72) Inventor: Hulkenberg, Antonius
c/o OCTROOIBUREAU ZOAN B.V. Apollolaan 151
NL-1077 AR Amsterdam(NL)

(74) Representative: Muis, Maarten, Drs. et al,
OCTROOIBUREAU ZOAN B.V. Apollolaan 151
NL-1077 AR Amsterdam(NL)

## (54) Phenyl piperazine derivatives having antiaggressive activity.

(57) The group of new phenylpiperazine derivatives of formula 3 of the formula sheet and the salts thereof have been found to have a strong antiaggressive activity. The compounds can be prepared in a manner known for analogous compounds and be processed to the usual compositions.

EP 0 089 089 A1

Phenyl piperazine derivatives having antiaggressive activity.

The invention relates to new phenyl piperazine derivatives, to the preparation of these compounds and to compositions comprising at least one of these compounds as the active substance.

Various phenyl piperazine derivatives with or without pharmacological properties are known.

The preparation of five compounds of formula 1 of the formula sheet, wherein R is the phenyl, p-chlorophenyl-, p-fluorophenyl-, p-methoxyphenyl or 3,5-dimethoxyphenyl group is known from Indian. J. Appl. Chem. (1972), 35, pp. 129-130 (which article is referred to in Chem. Abstr. 81, 120577 n).

These compounds have been prepared in the framework of an investigation into new anthelmintics.

Furthermore, an article in J. Med. Chem. 9 (1966) pp. 153-155 relates to compounds of formula 2, wherein $R_1$ may be the phenyl group or 4-chlorophenyl group, $R_2$ is, for example, hydrogen, methyl, ethyl, propyl, tert. butyl, phenyl or substituted phenyl and n is 1 or 2.

The compounds have been tested for various activities and in most of the cases proved to have no activity.

It has now been found that phenylpiperazine derivatives of formula 3 of the formula sheet, wherein $R_3$ is a trifluoromethyl group or a chlorine atom, $R_4$ is a hydrogen atom, methyl group or ethyl group, A is the group $-CH_2-$, $-CH_2CH_2-$, $CH_3-\underset{|}{C}H-CH_2-$ or $-CH_2-\underset{|}{C}H-CH_3$, X is the group $-CH_2-$, $>C=O$ or $>SO_2$, with the proviso that, when A comprises more than one C-atom, X cannot be $-CH_2-$; Y is the group $>C=O$ or $>SO_2$ with the proviso that, when X is the group $-CH_2-$, Y is the group $>SO_2$, and that X cannot be equal to Y; Z is the group $-NR_5R_6$, wherein $R_5$ and $R_6$ are hydrogen or an alkyl group having 1-4 C-atoms, or Z is an alkyl group having 1-4 C-atoms, the cyclohexyl group, cyclohexylmethyl group, cyclohexyloxymethyl group or one of the groups of formulae 4-10, wherein $R_7$ is hydrogen, 4-fluoro, 4-chloro, 4-methyl, 4-methoxy, 3-trifluoromethyl, 4-trifluoromethyl, 2,6-difluoro, 2,6-dichloro and m is an integer having the value 2-5, or Z is

a group $R_8O$-, wehrein $R_8$ is an alkyl group or cycloalkyl group having 1-6 C-atoms or an aralkyl radical the aryl group of which may be substituted by a group $R_7$, and the salts thereof, have a strong antiaggressive activity. Moreover, some compounds belonging to this group are readily analgetically active.

The antiaggressive activity of the compounds was measured in a test suitable for that purpose in isolated mice (Advances in Pharmacol. 5 (1967), 79). In this test, male albino mice were kept isolated for a period of 4 weeks and were then selected for the test on the basis of fighting behaviour present. The selection criterion is the occurrence of 3 or more fighting periods within 3 minutes after a mouse which has not been kept isolated has been placed in the cage of the mouse which has been kept isolated.

The compounds to be investigated were administered orally to the selected mice. 5 Mice were used per dosis. Sixty minutes after administration of the compounds to be examined, the animals were again evaluated for fighting behaviour. The compound to be investigated is inactive in the administered dosis when this time also 3 or more fighting periods were observed within 3 minutes after a mouse which has not been kept isolated was placed with the mouse which had been kept isolated. The $ED_{50}$-value in mg of active substance per kg of body weight was calculated from the results obtained.

The compounds according to the invention have an $ED_{50}$-value which is smaller than 10 mg/kg and for most compounds the $ED_{50}$-value is 1-5 mg/kg.

Due to the strong antiaggressive activity and the absence of undesired side effects, for example sympatholytic, dopaminolytic. muscle-relaxing and sedative properties, the compounds are excellently suitable for use in the treatment of intra- and extrapunitive behaviour and overt aggressive behaviour in man and animals.

For medical use in men is to be considered first of all the control of aggressive symptoms in psychiatric symptoms of a disease and serious forms of psychopathological aggression.

As a possibility of application in the veterinary field are to be considered above all those forms of aggression

which occur in transporting agricultural domestic animals and the mixing of groups of these animals.

The quantity, frequencé and mode of administration may differ for each individual case, also dependent on the nature and the severity of the disturbances. Generally, a dose from 5 to 500 mg and preferably from 25 to 150 mg daily will be suitable for humane application.

For veterinary applications the dose is preferably from 0.1 to 10 mg/kg of body weight.

The active compounds according to the invention and their salts can be processed according to known standard methods to compositions such as pills, tablets, coated tablets, capsules, powders, injection liquids and the like while using the conventional auxiliary substances such as solid and liquid carrier materials.

As examples of pharmaceutically acceptable acids with which the compounds according to the invention can form salts may be mentioned hydrochloric acid, sulphuric acid, nitric acid, citric acid, fumaric acid, maleic acid, tartaric acid, methanesulphonic acid, benzoic acid and the like.

The compounds of formula 3 and their salts can be prepared according to methods which are suitable for the synthesis of analogous compounds. Therefore the invention also relates to the preparation of the new compounds and the salts thereof.

The compounds of formula 3 can be obtained, for example, by reaction of a compound of the general formula 11 of the formula sheet with a compound of the formula $Cl-A-X-NR_4-Y-Z$, wherein $R_3$, $R_4$, A,X,Y and Z have the above meanings. This reaction is preferably carried out in an inert solvent, for example ethanol or acetonitrile at a temperature between 20°C and the boiling point of the solvent used, in the presence of an acid binder.

Compounds of formula 3 can also be obtained by reacting a compound of formula 12 with a compound of the formula Z-Y-Cl, in which formulae the symbols have the above meanings.

Furthermore, compounds of formula 3 can be obtained by reaction of a compound of formula 13 with a compound

of the formula $HNR_5R_6$ or $R_8OH$, wherein the symbols have the above meanings. This reaction is preferably carried out in an organic solvent, for example, ether, tetrahydrofuran, benzene, toluene or methylene chloride, at a temperature between $0^oC$ and the boiling point of the solvent used.

The compounds of formula 3 can furthermore be obtained by reaction of a compound of formula 14 with a compound of the formula $NH_2$-A-X-$NR_4$-Y-Z. This conversion is preferably carried out in a solvent, for example butanol, in the presence of an acid binder, for example $K_2CO_3$, at temperatures between room temperature and the boiling-point of the solvent.

In similar cirucumstances the compounds of formula 3 can be obtained by reaction of a compound of formula 15 with a compound of formula 16.

Finally, compounds of formula 3 can be obtained by reaction of a compound of formula 17 or an acid addition salt thereof, with a compound of the formula Z-Y-$NHR_4$.

The invention will be described in greater detail with reference to the following examples.

EXAMPLE I

N-[3-{4-(3-trifluoromethylphenyl)-1-piperazinyl}propionyl]sulfamide.

2.0 g (10.7 mmol) of 3-chloropropionylsulfamide, 2.2 g (10 mmol) of 3-trifluoromethylphenylpiperazine and 1.4 ml (10 mmol) of triethylamine were together refluxed for 1 hour with vigorous stirring in 50 ml of tetrahydrofuran. After cooling to room temperature, the precipitate formed was sucked off and washed well with ether. The product now crystallizing from the filtrate was recrystallized from methyl ethyl ketone, after which the title compound was obtained with a melting-point of 150.5-151$^oC$.

EXAMPLE II

N-[3-{4-(3-trifluoromethylphenyl)-1-piperazinyl}propionyl]benzenesulfonamide.

3.4 g (13,8 mmol) of chloropropionylbenzenesulfonamide, 2.9 g (12.8 mmol) of 3-trifluoromethylphenylpiperazine and 0.95 g (6.9 mmol) of potassium carbonate were together refluxed for 1 hour with vigorous stirring in 25 ml of acetonitrile. After cooling to room temperature, the resulting precipitate was sucked off and then washed with water. The material obtained in

this manner was recrystallized from acetonitrile, after which the title compound was obtained with a melting-point of 187-188°C.

EXAMPLE III

N-[2-{4-(3-trifluoromethylphenyl)-1-piperazinyl}ethyl] methanesulfonamide.

14.7 g (54 mmol) of 2-[4-(3-trifluoromethylphenyl)-1-piperazinyl] ethylamine, 6.3 g (54 mmol) of methanesulfonylchloride and 7.5 ml (54 mmol) of triethylamine were together refluxed for 1 hour with vigorous stirring in 120 ml of dry toluene. After cooling to room temperature the precipitate formed was sucked off. The filtrate was evaporated under reduced pressure and the residue was then purified chromatographically over silica gel with ethyl acetate as an eluent. The title compound obtained in this manner had a melting-point of 61.5 - 63.5°C.

CLAIMS:

1. Compounds of formula 3 of the formula sheet and salts thereof with pharmaceutically acceptable acids, in which formula

$R_3$ is a trifluoromethyl group or a chlorine atom,

$R_4$ is a hydrogen atom, methyl group or ethyl group,

A is the group $-CH_2-$, $-CH_2-CH_2-$, $CH_3-CH-CH_2-$ or $-CH_2-CH-CH_3$,

X is the group $-CH_2-$, $>C=O$ or $>SO_2$, with the proviso that, when A comprises more than one C-atom, X cannot be $-CH_2-$;

Y is the group $>C=O$ or $>SO_2$, with the proviso that, when X is the group $-CH_2-$, Y is the group $>SO_2$, and that X cannot be equal to Y,

Z is the group $-NR_5R_6$, wherein $R_5$ and $R_6$ are hydrogen or an alkyl group having 1-4 C-atoms, or Z is an alkyl group having 1-4 C-atoms, the cyclohexyl group, cyclohexylmethyl group, cyclohexyloxymethyl group or one of the groups of the formulae 4-10, wherein $R_7$ is hydrogen, 4-fluoro, 4-chloro, 4-methyl, 4-methoxy, 3-trifluoromethyl, 4-trifluoromethyl, 2,6-difluoro, 2,6-dichloro and $\underline{m}$ is an integer number having the value 2-5,

or Z is a group $R_8O$, wherein $R_8$ is an alkyl group or cycloalkyl group having 1-6 C-atoms or an aralkyl radical the aryl group of which may be substituted by a group $R_7$.

2. Pharmaceutical compositions, characterized in that they comprise at least one compound of formula 3 of the formula sheet as an active substance, in which the symbols have the meanings given in Claim 1, or a salt thereof with a pharmaceutically acceptable acid.

3. A method of preparing pharmaceutical compositions, characterized in that a compound of Claim 1 is brought into a form suitable for administration.

4. A method of preparing pharmaceutically active phenylpiperazine derivatives, characterized in that antiaggressive compounds of formula 3 of the formula sheet, wherein the symbols have the meanings given in Claim 1, and salts thereof with pharmaceutcially acceptable acids are prepared in a manner known for the synthesis of analogous compounds.

5. A method as claimed in Claim 4, characterized in that a compound of formula 11 of the formula sheet is converted with a compound of the formula $Cl-A-X-NR_4-Y-Z$, in which formulae the symbols have the meanings given in Claim 1.

6. A method as claimed in Claim 4, characterized in that a compound of formula 12 is converted with a compound of the formula Z-Y-Cl, in which formulae the symbols have the meanings given in Claim 1.

7. A method as claimed in Claim 4, characterized in that a compound of formula 13 of the formula sheet is converted with a compound of the formula $NHR_5R_6$ or $R_8OH$, in which formulae the symbols have the meanings given in Claim 1.

8. A method as claimed in Claim 4, characterized in that a compound of formula 14 of the formula sheet is converted with a compound of the formula $NH_2$-A-X-$NR_4$-Y-Z, wherein the symbols have the meanings given in Claim 1.

9. A method as claimed in Claim 4, characterized in that a compound of formula 15 is converted with a compound of formula 16 of the formula sheet, in which formulae the symbols have the meanings given in Claim 1.

10. A method as claimed in Claim 4, characterized in that a compound of formula 17 is converted with a compound of the formula Z-Y-$NHR_4$, in which formulae the symbols have the meanings given in Claim 1.

11. A method of controlling intrapunitive and extrapunitive behaviour and overt aggressive behaviour in man or animal, characterized in that an active quantity of a compound of formula 3 wherein the symbols have the meanings given in Claim 1, or a salt thereof with a pharmaceutically acceptable acid, is administered.

FORMULA SHEET

0089089

1. 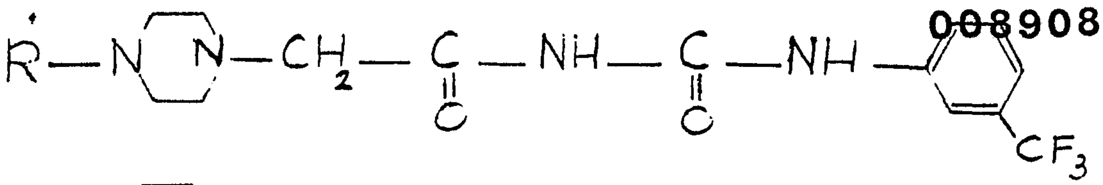

2. 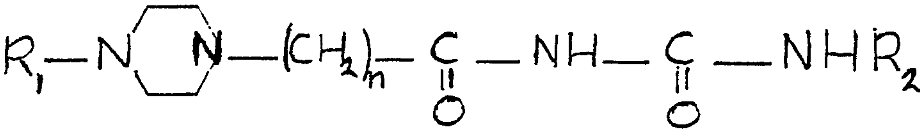

3. 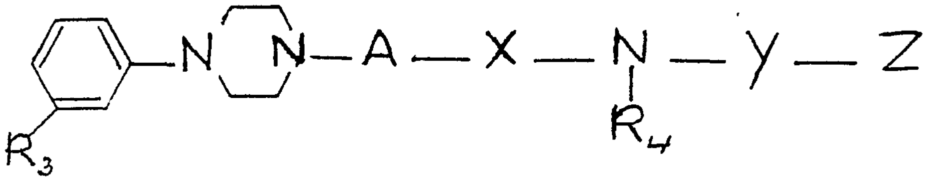

4. 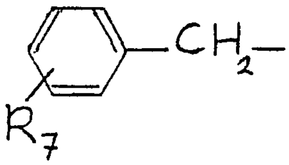

5. 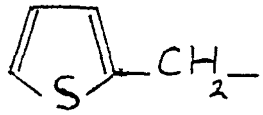

6. 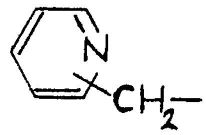

7. 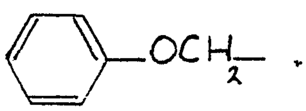

8. 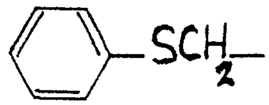

9. 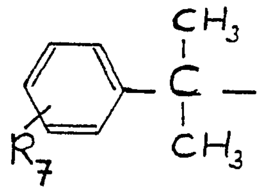

10. 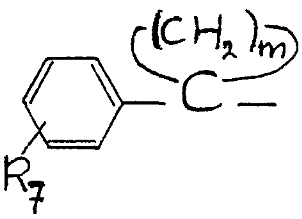

11. 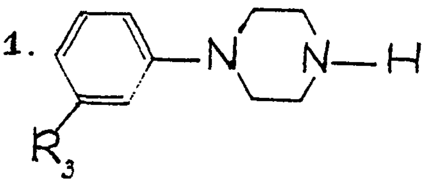

12. 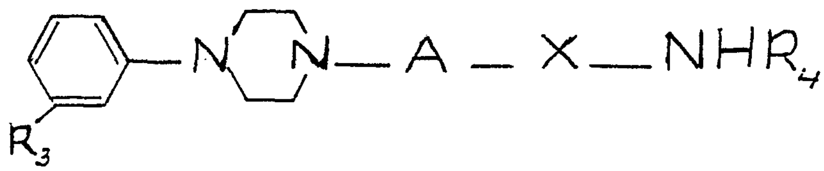

13. 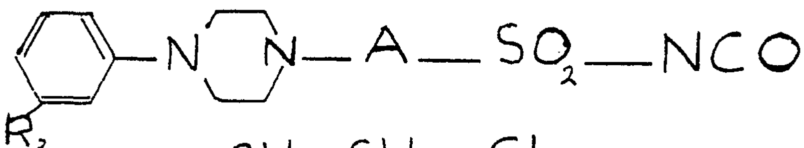

14. 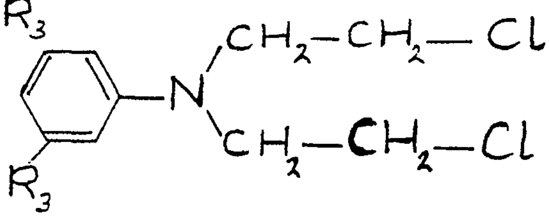

15. 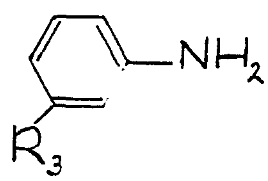

16. 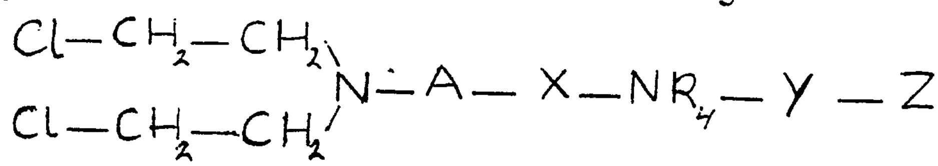

17. 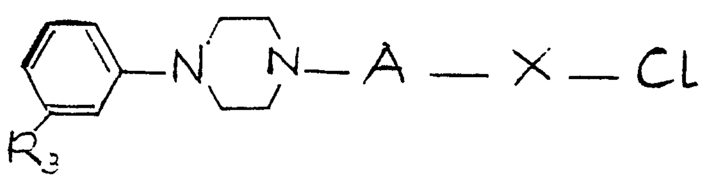

833c

European Patent Office

# EUROPEAN SEARCH REPORT

0089089

Application number

EP 83 20 0346

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | US-A-3 455 927 (J.J. LAFFERTY et al.) * Columns 1-2 * | 1-4,6, 11 | C 07 D 295/08 C 07 D 295/12 C 07 D 295/14 A 61 K 31/495 |
| | --- | | |
| Y | EP-A-0 021 592 (J.A. WUELFING) * Claims * | 1-4,11 | |
| | --- | | |
| Y | EUROPEAN JOURNAL OF MEDICINAL CHEMISTRY, vol. 12, no. 2, 1977, pages 173-176, FR. P.E. CROSS et al.: "Substituted trifluoromethyl phenyl piperazines as anorectic agents" * Page 174 * | 1-3,11 | |
| | --- | | |
| Y | FR-A-2 209 227 (PARCOR) * Claims * | 1-4,11 | TECHNICAL FIELDS SEARCHED (Int. Cl. 3) |
| | --- | | |
| P,Y | EP-A-0 048 044 (DUPHAR INTERNATIONAL RESEARCH) * Claims * | 1-4,11 | C 07 D 295/00 A 61 K 31/00 |
| | ----- | | |

The present search report has been drawn up for all claims

| Place of search | Date of completion of the search | Examiner |
|---|---|---|
| THE HAGUE | 07-06-1983 | MOREAU J.M. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document